# EUROPÄISCHE PATENTANMELDUNG

(11) **EP 0 657 425 A1**
(43) Veröffentlichungstag der Anmeldung: **14.06.1995**
(21) Anmeldenummer: 94118573.8
(22) Anmeldetag: 25.11.1994
(51) Int. Cl.: C07D 207/08, C07D 205/04, C07D 211/22, C07C 215/42

(54) **Verfahren zur Herstellung chiraler Aminoalkohole**

(30) Priorität: 07.12.1993 DE 4341605
(71) Anmelder: Degussa Aktiengesellschaft, D-60311 Frankfurt (DE)
(72) Erfinder: Dauelsberg, Christine, D-28205 Bremen (DE); Behnen, Willi, D-26129 Oldenburg (DE); Martens, Jürgen, Prof. Dr., D-26122 Oldenburg (DE)

(57) **Zusammenfassung**

2.1 Die bekannten Verfahren zur Herstellung homochiraler Aminoalkohole der Formel I 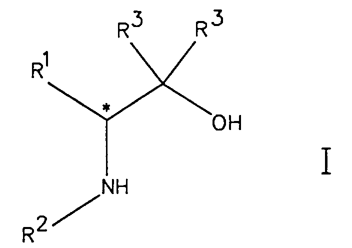
worin R¹, R², R³ und * die in der Beschreibung angegebene Bedeutung aufweisen, durch Umsetzung der entsprechenden Säure mit metallorganischen Verbindungen sind entweder aufwendig, ergeben eine geringe Ausbeute oder auch nur geringe Enantiomerenreinheit. Das neue Verfahren soll diese Nachteile vermeiden.

2.2 Erfindungsgemäß werden N-silylierte Aminosäuresilylester der Formel II 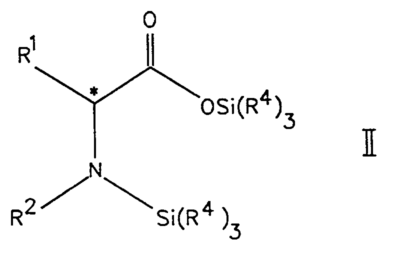
worin R¹, R², R⁴ und * die in der Beschreibung angegebene Bedeutung haben, mit metallorganischen Verbindungen, insbesondere Grignard-Reagenzien in hoher Ausbeute und Enantiomerenreinheit umgesetzt.

2.3 Chirale Katalysatoren in der enantioselektiven Reduktion unsymmetrischer Ketone.

## Beschreibung

Die Erfindung betrifft ein Verfahren zur Herstellung homochiraler Aminoalkohole der Formel I, 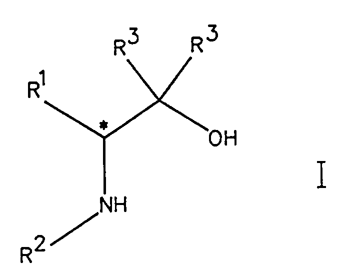
worin
R¹ ein geradkettiger, verzweigter oder zyklischer Alkyl-, Arylalkyl- oder Arylrest mit 1 - 20 C-Atomen ist, der im Falle einer Arylgruppe auch heteroatomsubstituiert sein kann,
R² ein Wasserstoffatom oder ein geradkettiger, verzweigter oder zyklischer Alkyl-, Arylalkyl- oder Arylrest mit 1 - 20 C-Atomen ist, wobei die Arylgruppe des Arylalkylrestes oder der Arylrest auch heteroatomsubstituiert sein können und wobei R² auch mit R¹ verbunden sein kann,
R³ entweder identische oder untereinander verbundene geradkettige, verzweigte oder zyklische Alkyl-, Arylalkyl- oder Arylreste mit 1 - 20 C-Atomen sind, wobei die Arylgruppe des Arylalkylrestes oder der Arylrest auch einen oder mehrere Alkyl-, Arylalkyl-, Aryl- oder Heteroatomsubstituenten aufweisen können oder an einem weiteren Arylring ankondensiert sein können und worin
* ein Asymmetriezentrum bedeutet, sowie deren Säureadditions-Salzen.

Homochirale Aminoalkohole sind beispielsweise als chirale Katalysatoren in der enantioselektiven Reduktion unsymmetrischer Ketone von Interesse, weil mit deren Hilfe die Erzeugung sekundärer Alkohole von hoher optischer Reinheit gelingen kann. Insbesondere sind hierfür auch zyklische Aminoalkohlole geeignet (DE-OS 36 09 152).

Ein bereits 1933 (Hoppe-Seylers Zeit. Physiol. Chem. 1933, 223, 43 - 52) erstmalig synthetisierter zyklischer Aminoalkohol erlangte aufgrund seiner hohen chiralen Katalysatorwirkung in jüngster Vergangenheit besonderes Interesse. Es handelt sich um den sog. Corey-Katalysator oder (S)-2-(Diphenylhydroxymethyl)pyrrolidin.

Allerdings zeigten die ersten Synthesen relativ geringe absolute Ausbeuten und geringe Enantiomerenreinheit. So lieferte die 1933 beschriebene Umsetzung von (S)-Prolinmethylesterhydrochlorid mit Phenylmagnesiumbromid insgesamt nur 26,4 % Ausbeute, bei einer Nacharbeitung (J. Org. Chem., Bd 56, 1991, S. 751 - 762 nur 20 % (S)-2-(Diphenylhydroxymethyl)pyrrolidin mit 80 % ee., und die 1965 bekanntgewordene (FR-A-3638) Umsetzung von (S)-Prolinethylester mit Phenylmagnesiumchlorid ergab ebenfalls nur 21 % (S)-2-(Diphenylhydroxymethyl)pyrrolidin, bezogen auf das eingesetzte Prolin.

Die Technik der Umsetzung von α-Iminocarbonsäurealkylestern mit Grignard-Verbindungen zur Gewinnung von chiralen Aminoalkoholen ist bis heute Gegenstand eingehender Untersuchungen.

So ist beispielsweise (Synthetic Communications 22, 2143 - 2153 (1992)) ausgehend von der (S)-Azetidincarbonsäure über deren Methylesterhydrochlorid mit Phenylmagnesiumbromid die der Corey-Verbindung analoge 4-Ring-Verbindung hergestellt worden.

Weiterhin ist es auch bekannt, chirale zyklische Aminoalkohole aus Grignard-Verbindungen und α-Iminocarbonsäure-N-carboxanhydriden (J. Org. Chem., Bd. 56, 1991, S. 751 - 762) herzustellen.

Ferner wurden Versuche unternommen, racemische zyklische Aminoalkohole zur Gewinnung von enantiomerenreinen zyklischen Aminoalkoholen einer Racematspaltung (J. Am. Chem. Soc., Bd. 109, 1987, S. 7925 - 7926) mit den Enantiomeren der O-Acetylmandelsäure zu unterwerfen.

Die genannten Verfahren ergeben oft nur mäßige Ausbeuten und machen es notwendig, das Grignard-Reagenz in großem Überschuß einzusetzen. Außerdem wurden bei der Umsetzung von (S)-Prolinalkylestern mit Grignard-Verbindungen die zyklischen Aminoalkohole in ungenügender Enantiomerenreinheit erhalten (J. Org. Chem., Bd. 56, 1991, S. 751 - 762). Schließlich sind Alkylester von α-Iminocarbonsäuren instabil und bilden leicht unter Abspaltung von Alkohol stabile zyklische Diketopiperazine, die nur schwer abzutrennen sind. Die Verwendung von α-Iminocarbonsäure-N-carboxanhydriden ist nachteilig, weil zu deren Herstellung das besonders giftige Phosgen notwendig und dieses nicht überall verfügbar ist.

Aus Tetrahedron, Vol. 49, No. 23, pp. 5127 - 5132, 1993, ist kürzlich ein Verfahren zur Herstellung von chiralem α,α-Diphenyl-2-pyrrolidinmethanol bekannt geworden, bei dem zur Umsetzung mit der Grignard-Verbindung sowohl die Carboxylgruppe des Prolins als Methylester als auch die Iminofunktion als N-Carbamat geschützt werden. Allerdings ist die Vorgehensweise recht aufwendig, weil entweder in zwei Schritten getrennt oder in einer Eintopfreaktion zunächst in einem ersten Schritt das S-Prolin-N-ethyl-carbamat und dann in einem zweiten Schritt der Methylester herzustellen sind. Wird die gesamte Reaktion in 4 einzelnen Schritten einschließlich einer Chromatographie durchgeführt, beträgt die Gesamtausbeute 65 %. Wird die Reaktion in einem vereinfachten Verfahren ohne Zwischenisolierung durchgeführt, wird keine Ausbeute angegeben.

Aus der DE-OS 36 09 152 kennt man Verfahren zur Herstellung optisch aktiver Prolinderivate, bei dem man entweder in einer zweistufigen Synthese S-Prolinester mit Grignard-Verbindungen in Gegenwart eines Verdünnungsmittels umsetzt oder in einer dreistufigen Synthese S-Prolinester in einer ersten Stufe mit Chlorameisensäurebenzylester in Gegenwart eines Säurebindemittels und in Gegenwart eines Verdünnungsmittels umsetzt, dann in einer zweiten Stufe die dabei entstehenden, am asymmetrischen substituierten Kohlenstoffatom S-konfigurierten Ester mit Grignard-Verbindungen umsetzt und schließlich in einer dritten Stufe die erhaltenen S-Prolin-Derivate mit Wasserstoff in Gegenwart eines Katalysators sowie in Gegenwart eines Verdünnungsmittels umsetzt.

Bei der in der DE-OS 36 09 152 beschriebenen "dreistufigen Variante" handelt es sich in Wirklichkeit um ein fünfstufiges Verfahren (Aminosäure → Ester → N-geschützter Ester → N-geschützter Alkohol → Alkoholhydrochlorid → Alkohol), das bei aufwendiger Verfahrensführung nur schlechte Ausbeuten liefert. Bei der zweistufigen Variante werden nachteilig sehr hohe Mengen Grignard-Reagenz eingesetzt (10 Äquivalente), während bei der "fünf-stufigen" Variante die Gesamtausbeute nur 37,7 % beträgt, insbesondere bedingt durch die zusätzlichen Verfahrensschritte für Einführung und Abspaltung der Schutzgruppe.

Alle bisher bekannt gewordenen Verfahren zur Herstellung homochiraler Aminoalkohole sind also auf die eine oder andere Weise nachteilig.

Aufgabe der vorliegenden Erfindung ist es daher, ein Verfahren zur Herstellung der in Formel I angegebenen Verbindungen zur Verfügung zu stellen, das es gestattet, homochirale Aminoalkohole, insbesondere zyklische Aminoalkohole, in hoher Ausbeute und Enantiomerenreinheit in wenigen einfachen Reaktionsschritten herzustellen, wobei gleichzeitig die erwünschten Endprodukte möglichst einfach zu isolieren sein sollen.

Gelöst wird die Aufgabe erfindungsgemäß durch ein Verfahren mit den im kennzeichnenden Teil des Anspruchs 1 angegebenen Maßnahmen.

Insbesondere dadurch, daß ein N-silylierter Aminosäuresilylester der Formel II 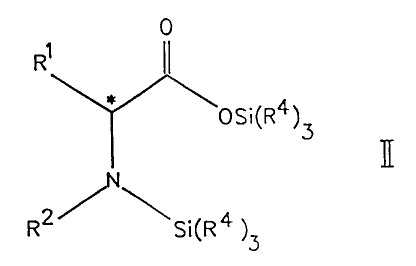
worin
R¹ ein geradkettiger, verzweigter oder zyklischer Alkyl-, Arylalkyl- oder Arylrest mit 1 - 20 C-Atomen ist, der im Falle einer Arylgruppe auch heteroatomsubstituiert sein kann,
R² ein Wasserstoffatom oder ein geradkettiger, verzweigter oder zyklischer Alkyl-, Arylalkyl- oder Arylrest mit 1 - C-Atomen ist, wobei die Arylgruppe des Arylalkylrestes oder der Arylrest auch heteroatomsubstituiert sein können und wobei R² auch mit R¹ verbunden sein kann,
R³ entweder identische oder untereinander verbundene geradkettige, verzweigte oder zyklische Alkyl-, Arylalkyl- oder Arylreste mit 1 - 20 C-Atomen sind, wobei die Arylgruppe des Arylalkylrestes oder der Arylrest auch einen oder mehrere Alkyl-, Arylalkyl-, Aryl- oder Heteroatomsubstituenten aufweisen können oder an einem weiteren Arylring ankondensiert sein können,
R⁴ eine Alkylgruppe mit 1 - 4 C-Atomen ist und worin
* ein Asymmetriezentrum bedeutet,
mit einer metallorganischen Verbindung, insbesondere einem Grignard-Reagenz, umgesetzt wird,
ist es auf sehr einfache Weise möglich, homochirale Aminoalkohole in hoher Ausbeute und mit hoher Reinheit des erwünschten Enantiomeren herzustellen.

Das erfindungsgemäße Verfahren kann grundsätzlich bei allen N-silylierten Aminosäuresilylestern der Formel II erfolgreich angewendet werden. Es dient jedoch besonders bevorzugt zur Herstellung enantiomerenreiner zyklischer Aminoalkohole aus enantiomerenreinen zyklischen N-silylierten-α-iminocarbonsäuresilylestern, da diese zyklischen Aminoalkohole als Katalysatoren in der enantioselektiven Reduktion von asymmetrischen Ketonen oft eine sehr viel größere Stereoselektivität aufweisen als die azyklischen, also geradkettigen oder verzweigten Aminoalkohole.

Zu den zyklischen Aminoalkoholen gehören allgemein die unter die Formel I fallenden Verbindungen, bei denen die Reste R² und R¹ miteinander verbunden sind. Besonders bevorzugte enantioselektive Katalysatoren sind jedoch die Verbindungen gemäß der Formel Ia, 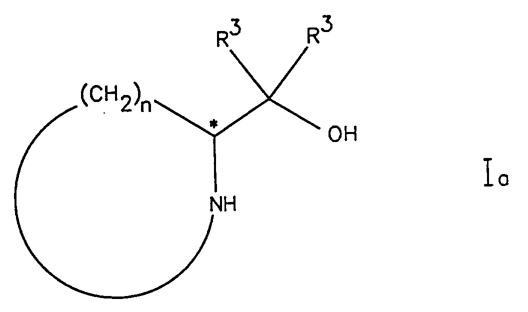
worin
R³ und * die in Formel I angebene Bedeutung haben und R¹ und R² aus Formel I miteinander verbunden sind und R¹ und R² aus Formel I unabhängig voneinander C₁-C₃-Alkylrest sind, die Summe von R¹ und R² aus Formel I jedoch nicht über C₄-Alkylrest hinausgeht, so daß in Formel Ia n 2, 3 oder 4 ist.

Diese besonders bevorzugten enantiomerenreinen zyklischen Aminoalkohole der Formel Ia sind nach dem erfindungsgemäßen Verfahren durch Umsetzung von enantiomerenreinen zyklischen N-silylierten-α-iminocarbonsäuresilylestern der Formel IIa 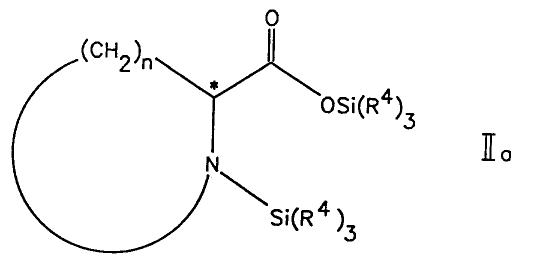
worin
R⁴ und * die bei Formel II angegebene Bedeutung haben, und n wie bei Formel Ia beschrieben 2, 3 oder 4 ist,
mit metallorganischen Verbindungen, insbesondere Grignard-Verbindungen, zugänglich.

Die N-silylierten Aminosäuresilylester der Formel II können vorteilhaft durch Umsetzung einer Aminosäure der Formel III 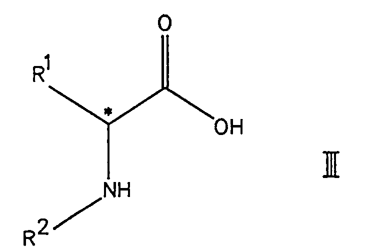
worin
R¹, R² und * die zu Formel I angebene Bedeutung haben,
mit einem Silylierungsmittel der Formel IV

X-Si(R⁴)₃ IV

worin
X eine Abgangsgruppe wie Cl, Br, J, NMe₂ oder NEt₂ ist und R⁴ die zu Formel I gegebene Bedeutung hat, hergestellt werden.

Die für die Darstellung der besonders bevorzugten zyklischen Aminoalkohole benötigten enantiomerenreinen N-silylierten-α-iminocarbonsäuretrimethylsilylester der Formel IIa sind vorteilhaft beispielsweise durch Umsetzung der jeweiligen enantiomerenreinen zyklischen α-Iminocarbonsäure der Formel IIIa 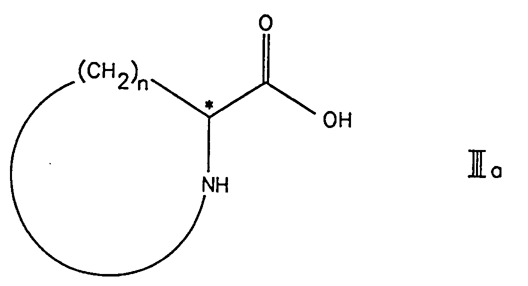
worin
* die in Formel Ia angegebene Bedeutung besitzt und n wie ebendort angegeben 2, 3 oder 4 ist unter Beachtung der dort angegebenen Bedingungen, mit einem Silylierungsmittel der Formel IV erhältlich. So ist z. B. die Verbindung der Formel IIa in hoher Ausbeute und Enantiomerenreinheit aus einer Verbindung der Formel IIIa mit N,N-Diethyltrimethylsilylamin (Chemische Berichte, Bd. 94, 1961, S. 1876 - 1878, Chemische Berichte, Bd. 99, 1966, S. 776 - 779) analog bekannter Methoden zugänglich. Darüber hinaus lassen sich enantiomerenreine α-Iminocarbonsäuren der Formeln III und IIIa bevorzugt auch mit Chlortrimethylsilan in Gegenwart einer Base zu den entsprechenden N-(Trimethylsilyl)-α-iminocarbonsäuretrimethylsilylestern (Journal of Organic Chemistry, Bd. 32, 1967, S. 1256 - 1257) umsetzen. Letztere Methode ist insbesondere aufgrund des relativ geringen Preises des Silylierungsreagenz von Vorteil. Ein weiterer großer Vorteil des erfindungsgemäßen Verfahrens besteht darin, daß ein einziges Reagenz zum Schutz sowohl des N-Atoms der Iminogruppe als auch der Carboxylgruppe eingesetzt werden kann, so daß die Schutzgruppeneinführung in einem einzigen Reaktionsschritt gelingt. Im Rahmen des erfindungsgemäßen Verfahrens werden die N-silylierten-α-iminocarbonsäuresilylester der Formel II oder IIa in die entsprechenden homochiralen Aminoalkohole überführt, indem sie mit metallorganischen Verbindungen reduziert werden.

Zu den im erfindungsgemäßen Reduktionsschritt bevorzugt einsetzbaren metallorganischen Verbindungen gehören u. a. Alkyl- und Aryllithiumverbindungen, wie Butyl- oder Phenyllithium, sowie vor allem Grignard-Verbindungen der Formel V

R³-Mg-Hal V

worin
R³ die in Formel I angebene Bedeutung hat und Hal für ein Halogen steht.

Bevorzugt steht R³ in Formel V für Phenyl, 4-Chlorphenyl, 4-Fluorphenyl, 4-Methylphenyl, 4-tert-Butylphenyl, 4-Methoxyphenyl, 3-Chlorphenyl, 3,5-Dimethylphenyl, 2-Naphthyl, Benzyl.

Wird eine Verbindung der Formel II oder IIa mit Grignard-Reagenzien umgesetzt, so resultiert nach der Hydrolyse das gewünschte homochirale Aminoalkoholsalz der Formel VI oder VIa 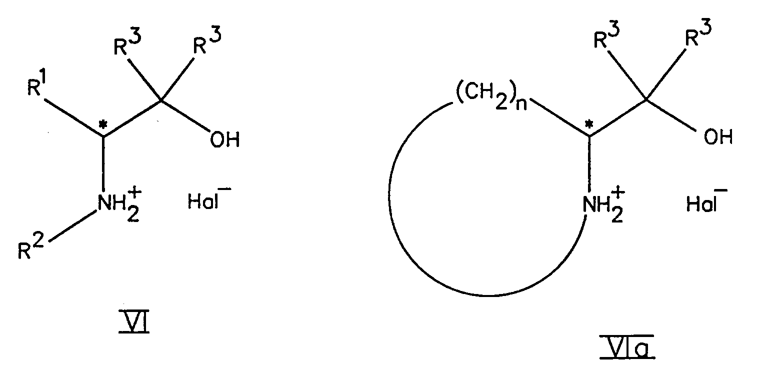
worin
R¹, R², R³ und * die in Formel II oder Ia angebene Bedeutung aufweisen und Hal für Halogen steht.

Das Grignard-Reagenz wird bevorzugt aus Magnesium und einer nur ein unter den Reaktionsbedingungen reaktives Halogenatom Y tragenden Verbindung der Formel VII

Y-R³ VII

erhalten,
worin
Y Chlor, Brom oder Iod ist und
R³ ein geradkettiger, verzweigter oder zyklischer Alkyl-, Arylalkyl- oder Arylrest mit 1 - 20 C-Atomen ist, wobei die Arylgruppe des Arylalkylrestes oder der Arylrest auch einen oder mehrere Alkyl-, Arylalkyl-, Aryl- oder Heteroatomsubsituenten aufweisen oder an einem weiteren Arylring ankondensiert sein kann.

Dabei ist es von Vorteil, wenn zum Erhalt der Grignard-Verbindung pro Äquivalent Magnesium mindestens ein Äquivalent der Verbindung der allgemeinen Formel VII eingesetzt wird.

Pro Äquivalent des Aminosäuresilylesters der Formel II oder IIa schließlich sollen bevorzugt mindestens zwei Äquivalente des Grignardreagenz der Formel VII eingesetzt werden.

Weiterhin bevorzugt kann das Grignard-Reagenz auch aus Magnesium und einer zwei unter den Reaktionsbedingungen reaktive Halogenatome Y tragenden Verbindung der Formel VIII

Y-(CₓH_{y}Z_{z})-Y VIII

erhalten werden,
worin
Y Chor, Brom oder Iod ist
und -(CₓH_{y}Z_{z})- eine beliebige, die beiden reaktiven Halogene Y verbindende Kohlenwasserstoffgruppe ist, die geradkettig, verzweigt, alizyklisch oder aromatisch ist und zusätzliche, unter den Reaktionsbedingungen nicht reaktive Substituenten Z wie Alkyl-, Alkoxy- oder Halogen aufweisen kann.

Wird eine Verbindung der Formel VIII zur Darstellung des Grignard-Reagenz verwendet, so hat es sich als günstig erwiesen, daß pro Äquivalent Magnesium nur ein halbes Äquivalent der Verbindung der Formel VIII eingesetzt wird.

Bei Umsetzung der Aminosäuresilylester der Formel II oder IIa wiederum hat es sich als besonders erstrebenswert herausgestellt, die Reaktion in einem Verdünnungsmittel, bevorzugt in einem Lösungsmittel durchzuführen.

Unter den Verdünnungs- und Lösungsmitteln kommen im wesentlichen alle unter den Reaktionsbedingungen gegenüber den Reaktanden inerten Lösungs- oder Verdünnungsmittel in Frage, bevorzugt werden jedoch aprotische Lösungsmittel, insbesondere solche mit Etherstruktur, wie Diethylether, Tetrahydrofuran oder 1,2-Dimethoxyethan.

Die erfindungsgemäßen Reaktionen lassen sich über einen weiten Temperaturbereich erfolgreich durchführen.

Es ist allerdings bevorzugt, daß die Umsetzung des Aminosäuresilylesters der Formel II mit dem Grignard-Reagenz in einem Lösungsmittel zwischen -70°C und der Siedetemperatur des verwendeten Lösungsmittels, bevorzugt zwischen 0°C und 20°C, durchgeführt wird.

Nach Beendigung des Reaktionsschrittes wird entweder das Salz des Aminoalkohols oder der freie Aminoalkohol aufgearbeitet. Hierbei kann man auf sehr einfache Weise, z. B. durch Behandlung mit einem basischen Ionenaustauscher oder mit einer geeigneten Base, das Aminoalkoholsalz in die freie Base des homochiralen cyclischen Aminoalkohols der Formel I oder Ia umwandeln.

Zur Aufarbeitung ist es insbesondere vorteilhaft, daß die Reaktionsmischung nach beendeter Reaktion abgekühlt wird, zur Hydrolyse in kalte Salzsäure gegossen, das ausgefallene Hydrochlorid des Aminoalkohols isoliert und in wäßriger Base gelöst, der freie Aminoalkohol aus dieser Lösung mit einem geeigneten organischen Lösungsmittel extrahiert und durch Eindampfen der organischen Phase isoliert und gegebenenfalls aus einem geeigneten Lösungsmittel umkristallisiert wird.

Die Homochiralität der erfindungsgemäß hergestellten Aminoalkohole der Formel I oder Ia ist von erheblicher praktischer Bedeutung, weil diese Verbindungen als stereoselektive Katalysatoren in enantioselektiven Synthesen eingesetzt werden.

### Beispiel 1

### (S)-2-(Diphenylhydroxymethyl)pyrrolidin

11.5 g (100 mmol) (S)-Prolin und 34.90 g (240 mmol) N,N-Diethyltrimethylsilylamin werden bei einer Ölbadtemperatur zwischen 110 und 120°C erhitzt. Dabei destilliert das entstehende Diethylamin kontinuierlich ab. Nach 6h ist die Reaktion beendet, da kein Diethylamin mehr entsteht. Der Rückstand wird bei 14 mbar/97°C destilliert. Man erhält 24.53 g (94.5 %) (S)-1-(Trimethylsilyl)pyrrolidin-2-carbonsäuretrimethylsilylester als sehr hydrolyseempfindliche klare Flüssigkeit.
¹H-NMR (CDCl₃): δ = 0.05, 0.26 [2s, 18H, OSi(CH₃)₃, NSi(CH₃)₃], 1.63 - 2.07 (m, 4H, 2 x H-3, 2 x H-4), 2.97 - 3.12 (m, 2H, H-5), 3.81 - 3.85 (dd, 1H, H-2). ¹³C-NMR (CDCl₃): δ = -0.9, -0.3 [OSi(CH₃)₃, NSi(CH₃)₃], 25.5 (C4), 31.7 (C3), 47.0 (C5), 61.6 (C2), 177.4 (C=O).

Eine Grignardverbindung bereitet aus 7.29 g (300 mmol) Magnesium, 47.1 g (300 mmol) Brombenzol in 350 ml abs. Ether wird bei 0-20°C unter Argon tropfenweise mit 19.46 g (75 mmol) (S)-1-(Trimethylsilyl)-pyrrolidin-2-carbonsäuretrimethylsilylester in 30 ml abs. Ether versetzt. Die Reaktionslösung wird 4h unter Rückfluß erhitzt, auf 0°C gekühlt und langsam unter Rühren in 450 ml 2N eiskalte Salzsäure (360 g Eis + 90 ml konz. HCl) gegossen. Der ausfallende weiße Feststoff wird abgesaugt, mit tert.-Butylmethylether und Wasser gewaschen und aus Methanol/tert.-Butylmethylether umkristallisiert. Man erhält in mehreren Fraktionen insgesamt 14.26 g (49.2 mmol) 65.6 % rohes (S)-2-(Diphenylhydroxymethyl)pyrolidin-Hydrochlorid.
Schmp.: 285-290°C (Zers.). ¹H-NMR (DMSO): δ = 1.76 - 1.88 (m, 4H, 2 x H-3, 2 x H-4), 3.09 - 3.25 (m, 2H, 2 x H-5), 4.85 - 5.01 (m, 1H, H-2), 6.50 (s, 1H, OH), 7.17 - 7.36, 7.52 - 7.54, 7.67 - 7.69 (3m, 10H, Ar-H), 8.30, 9.48 (2s, 2H, NH₂+). ¹³C-NMR (DMSO): δ = 24.0 (C4), 26.0 (C3), 46.8 (C5), 65.0 (C2), 77.2 [C(Ph)₂OH], 125.4, 125.9, 126.9, 127.2, 128.2, 128.4 (Ar-), 144.5, 145.0 (Ar, quart.-C).

Das rohe (S)-2-(Diphenylhydroxymethyl)pyrolidin-Hydrochlorid wird mit 100 ml 1N NaOH, 5 ml Triethylamin und 200 ml tert-Butylmethylether solange gerührt, bis sich zwei klare Phasen gebildet haben (ca.5h). Die Phasen werden getrennt, die wäßrige noch zweimal mit tert-Butylmethylether extrahiert und die vereinigten org. Phasen zweimal mit 20 %iger wäßriger NaCl-Lösung gewaschen. Die organische Phase wird getrocknet (MgSO₄), bei 40°C im Vakuum eingeengt und in heißem n-Heptan aufgenommen. In der Kälte fallen leicht gelbliche glasklare Kristalle aus. Man erhält 12.01 g (47.5 mmol) 63 % (S)-2-(Diphenylhydroxymethyl)pyrrolidin._ Schmp.: 76.5-78°C.
[α]_{D}²² = -58.4° (c= 3.0 MeOH) IR (KBr): n = 3400 - 3200 (OH), 3100 - 3000 cm⁻¹ s (Ar). ¹H-NMR (CDCl₃): δ = 1.49 - 1.78 (m, 4H, 2 x H-3, 2 x H-4), 2.83 - 3.00 (m, 2H, 2 x H-5), 4.20 (t, J = 7.40 Hz, 1H, H-2), 7.10 - 7.29, 7.46 - 7.58 (2m, 10H, Ar-H). ¹³C-NMR (CDCl₃): δ = 25.5 (C4), 26.3 (C3), 46.7 (C5), 64.5 (C2), 77.1 (C(Ph)₂OH), 125.5, 125.8, 126.3, 126.4, 127.9, 128.2 (Ar), 145.4, 148.2 (Ar, quart.-C). MS (CI, i-Butan): m/z = 254 (100 %, MH⁺), 255 (18 %), 70 (9 %), 236 (7 %).

| | | | | |
|---|---|---|---|---|
| C₁₇H₁₉NO (253.4): | Ber. | C80.60 | H7.56 | N5.53 |
| | Gef. | C80.54 | H7.89 | N5.67. |

### Beispiel 2

### (S)-2-(Diphenylhydroxymethyl)azetidin

Eine Grignardverbindung bereitet aus 2.43 g (100 mmol) Magnesium, 15.7 g (100 mmol) Brombenzol in 50 ml Diethylether wird mit einem Eisbad gekühlt und unter Argon werden 6.14 g (25 mmol) (S)-1-(Trimethylsilyl)-azetidin-2-carbonsäuretrimethylsilylester (Darstellung: Liebigs Annalen der Chemie 1990, Seiten 687-695) langsam zugetropft. Die Reaktionslösung wird 4h unter Rückfluß erhitzt, auf 0°C gekühlt und langsam unter Rühren in 150 ml 2N eiskalte Salzsäure (120 g Eis + 30 ml konz. HCl) gegossen. Der ausfallende weiße Feststoff wird abgesaugt und mit tert.-Butylmethylether und Wasser gewaschen.

Zur Darstellung des freien Aminoalkohols wird das rohe (S)-2-(Diphenylhydroxymethyl)azetidin-Hydrochlorid mit 50 ml 1N NaOH, 5 Tropfen Triethylamin und 100 ml Dichlormethan 1h gerührt. Die Phasen werden getrennt, die wäßrige nochmals mit Dichlormethan extrahiert und die vereinigten org. Phasen zweimal mit 20 %iger wäßriger NaCl-Lösung gewaschen. Die organische Phase wird getrocknet (MgSO₄), bei 40°C im Vakuum eingeengt und aus tert.-Butylmethylether/PE 40/60 umkristallisiert. Man erhält 4.97 g (20.8 mmol) 83 % (S)-2-(Diphenylhydroxymethyl)azetidin vom Schmp 114-116°C.
[α]_{D}²⁰ = -30.9 (c= 5.04 in CHCl₃). IR (KBr): ν = 3500 - 3150 (NH, OH), 3080 - 3000 (Ar). ¹H-NMR (CDCl₃): δ = 1.91 - 2.01 (m, 1H, 3-H), 2.34 - 2.46 (m, 1H, 3-H), 3.16 - 3.23 (m, 1H, 4-H), 3.53 - 3.61 (m, 1H, 4-H), 4.85 (t, J = 7.9 Hz, 1H, 2-H), 7.13 - 7.30, 7.37 - 7.42 (2m, 10H, Ar-H). ¹³C-NMR (CDCl₃): δ = 21.9 (1C, C-3), 42.3 (1C, C-4), 64.7 (1C, C-2), 76.6 (1C, C-α), 125.9, 126.1, 126.56, 126.6, 127.9, 128.0 (10C, Ar-C), 143.5, 146.5 (2C, Ar, 2 x quart.-C).

| | | | | |
|---|---|---|---|---|
| C₁₆H₁₇NO (239.3): | Ber.(%): | C 80.30 | H 7.16 | N 5.85 |
| | Gef.(%): | C 80.07 | H 7.13 | N 6.04. |

### Beispiel 3

Beispiel 1 wird wiederholt mit dem Unterschied, daß anstelle von Brombenzol Chlorbenzol eingesetzt wurde. Ausbeute an (S)-2-(Diphenylhydroxymethyl)pyrrolidin: 82% der Theorie.

| Elementaranalyse | | | | |
|---|---|---|---|---|
| C₁₇H₁₉NO (253.4): | Ber.(%) | C80.60 | H7.56 | N5.53 |
| | Gef.(%) | C80.56 | H7.83 | N5.62 |

Drehwert [α]_{D}²² = -58.9° (c= 3.0 in Methanol)

### Beispiel 4

Beispiel 1 wird wiederholt mit dem Unterschied, daß anstelle von 4-Brombenzol 4-Bromfluorbenzol eingesetzt wurde. Ausbeute an (S)-2-[Di-(4-fluorphenyl)hydroxymethyl]pyrrolidin: 90% der Theorie.

| Elementaranalyse | | | | |
|---|---|---|---|---|
| C₁₇H₁₇F₂NO (289.3): | Ber.(%) | C70.57 | H5.92 | N4.84 |
| | Gef.(%) | C70.48 | H5.89 | N4.69 |

Drehwert [α]_{D}²² = ⁻48.7° (c= 0.33 in Methanol)

### Beispiel 5

Beispiel 1 wird wiederholt mit dem Unterschied, daß anstelle von 4-Brombenzol 4-Bromchlorbenzol eingesetzt wurde. Ausbeute an (S)-2-[Di-(4-chlorphenyl)hydroxymethyl]pyrrolidin: 62% der Theorie.

| Elementaranalyse | | | | |
|---|---|---|---|---|
| C₁₇H₁₇Cl₂NO (322.2): | Ber.(%) | C63.37 | H5.32 | N4.35 |
| | Gef.(%) | C63.28 | H5.27 | N4.31 |

Drehwert [α]_{D}²² = -37.9° (c= 0.34 in Methanol)

### Beispiel 6

Beispiel 1 wird wiederholt mit dem Unterschied, daß anstelle von 4-Brombenzol 4-Bromtoluol eingesetzt wurde. Ausbeute an (S)-2-[Di-(4-methylphenyl)hydroxymethyl]pyrrolidin: 72% der Theorie.

| Elementaranalyse | | | | |
|---|---|---|---|---|
| C₁₉H₂₃NO (281.4): | Ber.(%) | C81.10 | H8.24 | N4.98 |
| | Gef.(%) | C80.92 | H8.35 | N4.89 |

Drehwert [α]_{D}²² = -43.9° (c= 0.31 in Methanol)

### Beispiel 7

Beispiel 1 wird wiederholt mit dem Unterschied, daß anstelle von 4-Brombenzol 4-(tert-Butyl)brombenzol eingesetzt wurde. Ausbeute an (S)-2-{Di-[4-(tert-butyl)phenyl]hydroxymethyl}pyrrolidin: 54% der Theorie.

| Elementaranalyse | | | | |
|---|---|---|---|---|
| C₂₅H₃₅NO (365.6): | Ber.(%) | C82.14 | H9.65 | N3.83 |
| | Gef.(%) | C82.09 | H9.39 | N3.64 |

Drehwert [α]_{D}²² = -26.0° (c= 0.40 in Methanol)

### Beispiel 8

Beispiel 1 wird wiederholt mit dem Unterschied, daß anstelle von 4-Brombenzol 4-Bromanisol eingesetzt wurde. Ausbeute an (S)-2-[Di-(4-methoxyphenyl)hydroxymethyl]pyrrolidin: 72% der Theorie.

| Elementaranalyse | | | | |
|---|---|---|---|---|
| C₁₉H₂₃NO₃ (313.4): | Ber.(%) | C72.82 | H7.40 | N4.47 |
| | Gef.(%) | C72.90 | H7.34 | N4.51 |

Drehwert [α]_{D}²² = -44.9° (c= 0.61 in Methanol)

### Beispiel 9

Beispiel 1 wird wiederholt mit dem Unterschied, daß anstelle von 4-Brombenzol 3-Bromchlorbenzol eingesetzt wurde. Ausbeute an (S)-2-[Di-(3-chlorphenyl)hydroxymethyl]pyrrolidin: 70% der Theorie.

| Elementaranalyse | | | | |
|---|---|---|---|---|
| C₁₇H₁₇Cl₂NO (322.2): | Ber.(%) | C63.37 | H5.32 | N4.35 |
| | Gef.(%) | C63.48 | H5.19 | N4.39 |

Drehwert [α]_{D}²² = -50.1° (c= 0.80 in Methanol)

### Beispiel 10

Beispiel 1 wird wiederholt mit dem Unterschied, daß anstelle von 4-Brombenzol 3,5-Dimethylbrombenzol eingesetzt wurde. Ausbeute an (S)-2-{Di-[3,5-dimethylphenyl]hydroxymethyl}pyrrolidin: 54% der Theorie.

| Elementaranalyse | | | | |
|---|---|---|---|---|
| C₂₁H₂₇NO (365.6): | Ber.(%) | C81.51 | H8.79 | N4.53 |
| | Gef.(%) | C81.40 | H8.69 | N4.62 |

Drehwert [α]_{D}²² = -64.0° (c= 0.32 in Methanol)

### Beispiel 11

Beispiel 1 wird wiederholt mit dem Unterschied, daß anstelle von Brombenzol 2-Naphthylbromid eingesetzt wurde. Ausbeute an (S)-2-[Di-(2-naphthyl)hydroxymethyl]pyrrolidin: 72% der Theorie.

| Elementaranalyse | | | | |
|---|---|---|---|---|
| C₂₅H₂₃NO (353.5): | Ber.(%) | C84.95 | H6.56 | N3.96 |
| | Gef.(%) | C84.76 | H6.83 | N4.04 |

Drehwert [α]_{D}²² = -132° (c= 2.56 in Chloroform)

### Beispiel 12

Beispiel 1 wird wiederholt mit dem Unterschied, daß anstelle von 4-Brombenzol Benzylbromid eingesetzt wurde. Ausbeute an (S)-2-[Dibenzylhydroxymethyl]pyrrolidin: 73% der Theorie.

| Elementaranalyse | | | | |
|---|---|---|---|---|
| C₁₉H₂₃NO (281.4): | Ber.(%) | C81.10 | H8.24 | N4.98 |
| | Gef.(%) | C81.22 | H8.36 | N4.87 |

Drehwert [α]_{D}²² = +10.2° (c= 0.55 in Methanol)

### Beispiel 13

Beispiel 2 wird wiederholt mit dem Unterschied, daß anstelle von (S)-1-(Trimethylsilyl)-azetidin-2-carbonsäuretrimethylsilylester (S)-1-(Trimethylsilyl)pipecolin-2-carbonsäuretrimethylsilylester eingesetzt wurde. Ausbeute an (S)-2-[Diphenylhydroxymethyl]pipecolin: 72% der Theorie.

| Elementaranalyse | | | | |
|---|---|---|---|---|
| C₁₈H₂₁NO (267.4): | Ber.(%) | C80.86 | H7.92 | N5.24 |
| | Gef.(%) | C80.92 | H8.15 | N4.98 |

Drehwert [α]_{D}²² = -58.9° (c= 2.02 in Methanol)

### Beispiel 14

Beispiel 1 wird wiederholt mit dem Unterschied, daß anstelle von (S)-Prolin (R)-Prolin eingesetzt wurde. Ausbeute an (R)-2-[Diphenylhydroxymethyl]pyrrolidin: 74% der Theorie.

| Elementaranalyse | | | | |
|---|---|---|---|---|
| C₁₇H₁₉NO (253.4): | Ber. | C80.60 | H7.56 | N5.53 |
| | Gef. | C80.72 | H7.69 | N5.48 |

Drehwert [α]_{D}²² = +58.9° (c= 3.0 in Methanol)

### Beispiel 15

### Darstellung von (S)-1-(Trimethylsilyl)pyrrolidin-2-carbonsäuretrimethylsilylester

Eine Suspension aus 23.02 g (200 mmol) Prolin, 86.91 g (800 mmol) Chlortrimethylsilan und 81.01 g (800 mmol) Triethylamin in 100 ml absolutem Ether wird bei Raumtemperatur 6h intensiv gerührt. Die weiße Suspension wird mittels einer Umkehrfritte filtriert und der abgetrennte Feststoff mit absoluten Ether gewaschen. Der auf diese Weise hergestellte mit Chlortrimethylsilan und Triethylamin verunreinigte (S)-1-(Trimethylsilyl)pyrrolidin-2-carbonsäuretrimethylsilylester wird zur Reinigung destilliert. Bei 10 mbar/ 94°C erhält man 33.87 g (65 %) (S)-1-(Trimethylsilyl)pyrrolidin-2-carbonsäuretrimethylsilylester als klare Flüssigkeit.

¹H-NMR (CDCl₃): δ = 0.05, 0.26 [2s, 18H, OSi(CH₃)₃, NSi(CH₃)₃], 1.63 - 2.07 (m, 4H, 2 x H-3, 2 x H-4), 2.97 - 3.12 (m, 2H, H-5), 3.81 - 3.85 (dd, 1H, H-2). ¹³C-NMR (CDCl₃): δ = -0.9, -0.3 [OSi(CH₃)₃, NSi(CH₃)₃], 25.5 (C4), 31.7 (C3), 47.0 (C5), 61.6 (C2), 177.4 (C=O).

Eine Grignardverbindung bereitet aus 14.57 g (600 mmol) Magnesium, 94.21 g (600 mmol) Brombenzol in 350 ml abs. Ether wird bei 0-20°C unter Argon tropfenweise mit dem oben hergestellten (S)-1-(Trimethylsilyl)pyrrolidin-2-carbonsäuretrimethylsilylester versetzt. Die Reaktionslösung wird 4h unter Rückfluß erhitzt, auf 0°C gekühlt und langsam unter Rühren in 450 ml 2N eiskalte Salzsäure (360 g Eis + 90 ml konz. HCl) gegossen. Der ausfallende weiße Feststoff wird abgesaugt und mit tert.-Butylmethylether und Wasser gewaschen. Das rohe Hydrochlorid wird mit 150 ml 1N NaOH, 5 ml Triethylamin und 300 ml tert-Butylmethylether solange gerührt, bis sich zwei klare Phasen gebildet haben (ca.3h). Die Phasen werden getrennt, die wäßrige noch zweimal mit tert-Butylmethylether extrahiert und die vereinigten org. Phasen zweimal mit 20 %iger wäßriger NaCl-Lösung gewaschen.

Die organische Phase wird getrocknet (MgSO₄), bei 40°C im Vakuum eingeengt und in heißem n-Heptan aufgenommen. In der Kälte fallen leicht gelbliche glasklare Kristalle aus. Man erhält 22.04 g (87 mmol) 67 % (S)-2-(Diphenylhydroxymethyl)pyrrolidin.

Gesamtausbeute: 44 % bezogen auf eingesetzte 200 mmol Prolin._
Schmp.: 75-76°C. [α]_{D}²² = -59.11° (c= 3.03 MeOH). IR (KBr): n = 3400 - 3200 (OH), 3100 - 3000 cm⁻¹ s (Ar). ¹H-NMR (CDCl₃): δ = 1.49 - 1.78 (m, 4H, 2 x H-3, 2 x H-4), 2.83 - 3.00 (m, 2H, 2 x H-5), 4.20 (t, J = 7.40 Hz, 1H, H-2), 7.10 - 7.29, 7.46 - 7.58 (2m, 10H, Ar-H). ¹³C-NMR (CDCl₃): δ = 25.5 (C4), 26.3 (C3), 46.7 (C5), 64.5 (C2), 77.1 (C(Ph)₂OH), 125.5, 125.8, 126.3, 126.4, 127.9, 128.2 (Ar), 145.4, 148.2 (Ar, quart.-C).

### Beispiel 16

### Umsetzung von (R)-Phenylglycin mit N,N-Diethyltrimethylsilylamin zum N-(Trimethylsilyl)-(R)-phenylglycintrimethylsilylester

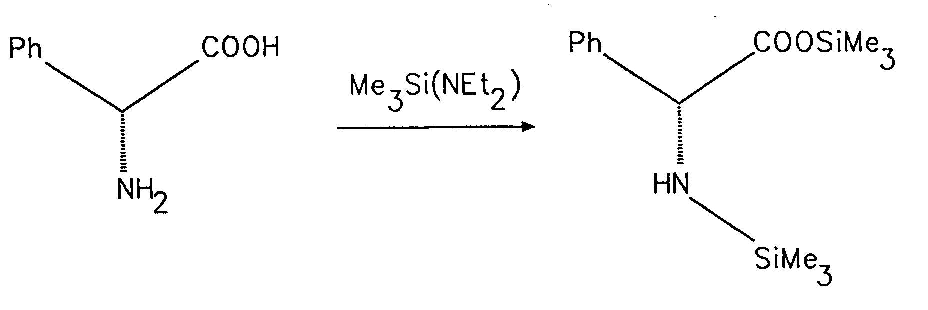

In einem ausgeheizten 100 ml Dreihalskolben mit Rückflußkühler wird unter Schutzgasatmosphäre 3.95 g (39.4 mmol) (R)-Phenylglycin und 20 g (138 mmol) N,N-Diethyltrimethylsilylamin gegeben. Die Suspension wird 4 h erhitzt (Badtemperatur 140°C), dabei löst sich die Aminosäure. Um das gebildete Diamin abzudestillieren, wird der Rückflußkühler gegen einen Liebigkühler ausgetauscht. Anschließend wird Vakuum gezogen, zuerst mit der Membranpumpe, dann mit der Drehschieberölpumpe, wobei die Lösung heftig schäumt. Die Destillation des gebildeten Silyls erfolgt bei 112°C und 2 mbar. Man erhält 15.4 g (40 %) des leicht gelblichen Destillates, das sehr hydrolyseempfindlich ist. Die Auswertung der NMR-Spektren zeigt, daß am Stickstoffatom nur eine Monosilylierung stattgefunden hat.

¹H-NMR (CDCl₃): δ = 0.02 und 0.22 (s, 9H, Si(CH₃)₃); 1.89 (d, 1H, H-2); 4.60 (s, 1H, NH); 7.31 (m, 5H, Ar-H). ¹³C-NMR (CDCl₃): δ = -0.58 und -0.03 (Si(CH₃)₃); 60.1 (C-2); 126.7-128.2 (Ar-C); 142.1 (Ar-C, ipso-C); 174.4 (COSi).

### (R)-1-(1'-amino-1'phenylmethyl)-cyclopentanol

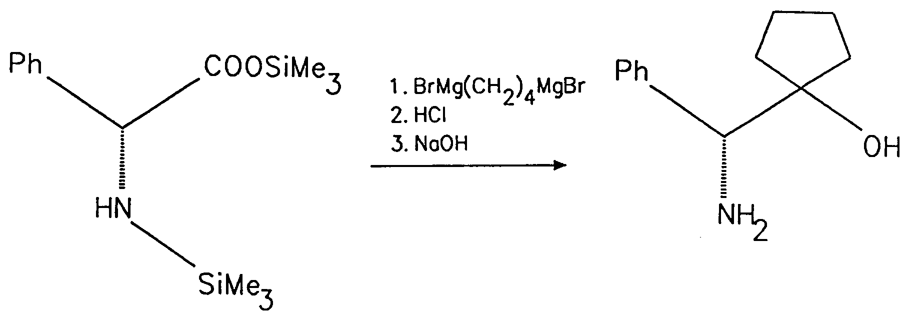

3.9 g (160 mmol) Magnesiumspäne werden in 50 mol abs. Diethylether vorgelegt. Von 9.5 ml (80 mmol) 1,4-Dibrombutan wird etwa 1/10 zu den Magnesiumspänen gegeben. Die Reaktion springt nach Zugabe von einigen Tropfen Brom an. Das restliche Alkylbromid wird in 250 ml abs. Diethylether so zugetropft, daß die Lösung leicht siedet. Beim anschließenden einstündigen Erhitzen unter Rückfluß löst sich das Magnesium fast völlig auf. Für die Zugabe des Silyls wird die dunkelgraue Lösung auf 10°C gekühlt und 7 g (24.0 mmol) des oben hergestellten N-(Trimethylsilyl)-(R)-phenylglycintrimethylsilylesters gelöst in 20 ml Diethylether langsam zugetropft. Nach der Zugabe wird die Lösung langsam auf Raumtemperatur erwärmt und 4 h unter Rückfluß erhitzt. Zur Hydrolyse wird die Lösung auf 15°C gekühlt und langsam in 320 g verd. Salzsäure-Lösung (12 ml konz. Salzsäure und 310 g Eis) gegossen. Um nicht gelöstes Magnesium abzutrennen, wird im Anschluß an die Hydrolyse sofort filtriert. Das gelbliche Filtrat wird unter vermindertem Druck eingeengt, bis der Diethylether abgetrennt ist. Der wäßrige Rückstand wird viermal mit Petrolether extrahiert und die wäßrige Phase weitgehend eingeengt. Das zurückbleibende öl wird fünfmal intensiv mit 50 ml CH₂Cl₂ extrahiert und die vereinigten organischen Phasen anschließend zweimal mit ges. Natriumchlorid-Lösung gewaschen. Die CH₂Cl₂-Phase wird mit 40 ml 2H NaOH-Lösung versetzt und die Lösung 24 h gerührt. Anschließend werden die Phasen getrennt und die wäßrige Phase zweimal mit CH₂Cl₂ extrahiert. Die vereinigten organischen Phasen werden einmal mit ges. Natriumchlorid-Lösung gewaschen und mit MgSO₄ getrocknet. Nach Abtrennen des Lösungsmittels bei vermindertem Druck verbleiben 2.67 g (56 %) eines Öls, das im Gefrierschrank langsam kristallisiert. Die Umkristallisation erfolgt aus Diethylether/Hexan und man erhält den Aminoalkohol in Form beiger Nadeln.

Ausbeute: 2.1 g (44 %).
Schmp.: 82 - 84°C.
[α]_{D}²⁰ = -23.8 (c = 0.998, CHCl₃).
IR (KBr): v = 3400 - 3200 (NH, OH); 2930 s (CH); 1470 cm⁻¹ (CH). ¹H-NMR (CDCl₃): d = 1.08 - 1.72 (m, 8H, Cyclop. H); 3.78 (s, 1H, H-1'); 7.12 - 7.23 (m, 5H, Ar-H). ¹³C-NMR (CDCl₃): d = 23.99 und 23.48 (Cyclop. C-3 und Cyclop. C-4); 34.93 und 36.05, (Cyclop. C-2 und Cyclop. C-5); 63.06 (C-1'); 127.0 - 127.8 (Ar-C); 142.6 (Ar, 1 x ipso-C).
MS (CI Isobutan): m/z(%) = 192 [MH⁺, 100 %].

| | | | | |
|---|---|---|---|---|
| C₁₂H₁₇N₁O₁ (191.3): | Ber. | C 75.35 | H 8.96 | N 7.32 |
| | Gef. | C 75.40 | H 9.04 | N 7.32 |

Im folgenden wird zur Verdeutlichung der breiten Anwendbarkeit und der Überlegenheit des erfindungsgemäßen Verfahrens eine Ausbeuteliste für die Beispiele 1 - 16 aufgeführt, aus der sich leicht die Gesamtausbeute für jedes der einzelnen Beispiele entnehmen läßt.

| **Beispiel** | **Schritt 1 (Silylester)** | **Schritte 2 + 3 (Alkohol)** | **Gesamtausbeute** |
|---|---|---|---|
| 1 | 94,5 % | 63 % | 59,5 % |
| 2 | 92 % (Lit.) | 83 % | 76,4 % |
| 3 | 94,5 % | 82 % | 77,5 % |
| 4 | 94,5 % | 90 % | 85,1 % |
| 5 | 94,5 % | 62 % | 58,6 % |
| 6 | 94,5 % | 72 % | 68,0 % |
| 7 | 94,5 % | 54 % | 51,0 % |
| 8 | 94,5 % | 72 % | 68,0 % |
| 9 | 94,5 % | 70 % | 66,2 % |
| 10 | 94,5 % | 54 % | 51,0 % |
| 11 | 94,5 % | 72 % | 68,0 % |
| 12 | 94,5 % | 73 % | 69,0 % |
| 13 | - | 72 % | - |
| 14 | 94,5 % | 74 % | 69,9 % |
| 15 | 65 % | 67 % | 43,6 % |
| 16 | 40 % | 44 % | 17,6 % |

## Patentansprüche

1. Verfahren zur Herstellung von homochiralen Aminoalkoholen der Formel I 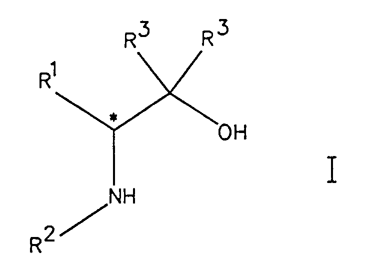 worin
R¹ ein geradkettiger, verzweigter oder cyclischer Alkyl-, Arylalkyl- oder Arylrest mit 1 - 20 C-Atomen ist, der im Falle einer Arylgruppe auch heteroatomsubstituiert sein kann,
R² ein Wasserstoffatom oder ein geradkettiger, verzweigter oder cyclischer Alkyl-, Arylalkyl- oder Arylrest mit 1 - 20 C-Atomen ist, wobei die Arylgruppe des Arylalkylrestes oder der Arylrest auch heteroatomsubstituiert sein können und wobei R² auch mit R¹ verbunden sein kann,
R³ entweder identische oder untereinander verbundene geradkettige, verzweigte oder cyclische Alkyl-, Arylalkyl- oder Arylreste mit 1 - 20 C-Atomen sind, wobei die Arylgruppe des Arylalkylrestes oder der Arylrest auch einen oder mehrere Alkyl-, Arylalkyl-, Aryl- oder Heteroatomsubstituenten aufweisen können oder an einem weiteren Arylring ankondensiert sein können und worin
* ein Asymmetriezentrum bedeutet, sowie deren Säureadditions-Salzen,
**dadurch gekennzeichnet,**
daß ein N-silylierter Aminosäuresilylester der Formel II 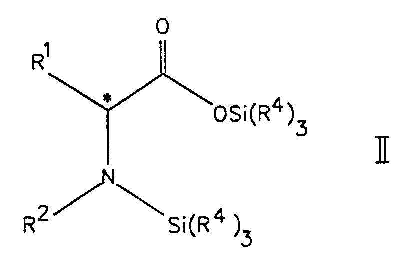 worin
R¹, R² und * die oben angegebenen Bedeutungen haben und R⁴ eine Alkylgruppe mit 1 - 4 C-Atomen ist,
mit einem metallorganischen Reagenz umgesetzt wird.

2. Verfahren nach Anspruch 1,
**dadurch gekennzeichnet,**
daß der N-silylierte Aminosäuresilylester der Formel II durch Umsetzung einer Aminosäure der Formel III, 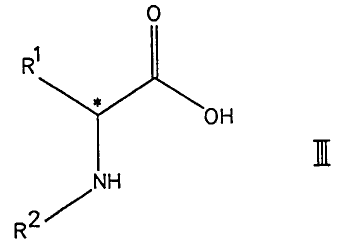 worin
R¹, R² und * die oben angegebenen Bedeutungen haben, mit einem Silylierungsmittel der Formel IV
X-Si(R⁴)₃ IV
worin
X eine Abgangsgruppe wie Cl, Br, I, NMe₂ oder NEt₂ ist und
R⁴ die oben angegebene Bedeutung hat, hergestellt wird.

3. Verfahren nach Anspruch 1 oder 2,
**dadurch gekennzeichnet,**
daß es sich bei dem metallorganischen Reagenz um ein Grignard-Reagenz handelt, das aus Magnesium und einer nur ein unter den Reaktionsbedingungen reaktives Halogenatom Y tragenden Verbindung der Formel VII
Y-R³ VII
erhalten wird,
worin
Y Chlor, Brom oder Iod ist und
R³ ein geradkettiger, verzweigter oder zyklischer Alkyl-, Arylalkyl- oder Arylrest mit 1 - 20 C-Atomen ist, wobei die Arylgruppe des Arylalkylrestes oder der Arylrest auch einen oder mehrere Alkyl-, Arylalkyl-, Aryl- oder Heteroatomsubsituenten aufweisen oder an einem weiteren Arylring ankondensiert sein kann.

4. Verfahren nach Anspruch 3,
**dadurch gekennzeichnet,**
daß pro Äquivalent Magnesium mindestens ein Äquivalent der Verbindung der allgemeinen Formel VII eingesetzt wird.

5. Verfahren nach einem der vorhergehenden Ansprüche,
**dadurch gekennzeichnet,**
daß pro Äquivalent des Aminosäuresilylesters mindestens zwei Äquivalente des Grignardreagenzes VII eingesetzt werden.

6. Verfahren nach Anspruch 1 oder 2,
**dadurch gekennzeichnet,**
daß es sich bei dem metallorganischen Reagenz um ein Grignard-Reagenz handelt, das aus Magnesium und einer zwei unter den Reaktionsbedingungen reaktive Halogenatome Y tragenden Verbindung der Formel VIII
Y-(CₓH_{y}Z_{z})-Y VIII
erhalten wird,
worin
Y Chor, Brom oder Iod ist
und -(CₓH_{y}Z_{z})- eine beliebige, die beiden reaktiven Halogene Y verbindende Kohlenwasserstoffgruppe ist, die geradkettig, verzweigt, alizyklisch oder aromatisch ist und zusätzliche, unter den Reaktionsbedingungen nicht reaktive Substituenten Z wie Alkyl-, Alkoxy- oder Halogen aufweisen kann.

7. Verfahren nach Anspruch 6,
**dadurch gekennzeichnet,**
daß pro Äquivalent Magnesium nur ein halbes Äquivalent der Verbindung der Formel VIII eingesetzt wird.

8. Verfahren nach einem der vorhergehenden Ansprüche,
**dadurch gekennzeichnet,**
daß die Umsetzung des Aminosäuresilylesters der Formel II mit dem Grignard-Reagenz in einem Verdünnungsmittel durchgeführt wird.

9. Verfahren nach Anspruch 8,
**dadurch gekennzeichnet,**
daß als Verdünnungsmittel eines mit Etherstruktur verwendet wird.

10. Verfahren nach einem der vorhergehenden Ansprüche,
**dadurch gekennzeichnet,**
daß die Umsetzung des Aminosäuresilylesters der Formel II mit dem Grignard-Reagenz in einem Lösungsmittel zwischen -70°C und der Siedetemperatur des verwendeten Lösungsmittels, bevorzugt zwischen 0°C und 20°C, durchgeführt wird.

11. Verfahren nach einem der vorhergehenden Ansprüche,
**dadurch gekennzeichnet,**
daß die Reaktionsmischung nach beendeter Reaktion abgekühlt wird, zur Hydrolyse in kalte Salzsäure gegossen, das ausgefallene Hydrochlorid des Aminoalkohols isoliert und in wäßriger Base gelöst, der freie Aminoalkohol aus dieser Lösung mit einem geeigneten organischen Lösungsmittel extrahiert und durch Eindampfen der organischen Phase isoliert und gegebenenfalls aus einem geeigneten Lösungsmittel umkristallisiert wird.
